(19) Europäisches Patentamt
European Patent Office
Office européen des brevets

(11) Numéro de publication: **0 152 310**
**B1**

(12) **FASCICULE DE BREVET EUROPÉEN**

(45) Date de publication du fascicule du brevet: **22.07.87**

(51) Int. Cl.⁴: **C 07 C 85/20**, C 07 C 87/60, C 07 B 39/00

(21) Numéro de dépôt: **85400013.0**

(22) Date de dépôt: **07.01.85**

(54) **Procédé de préparation de trifluorométhyl anilines.**

(30) Priorité: **20.01.84 FR 8400848**

(43) Date de publication de la demande:
**21.08.85 Bulletin 85/34**

(45) Mention de la délivrance du brevet:
**22.07.87 Bulletin 87/30**

(84) Etats contractants désignés:
**AT BE CH DE FR GB IT LI LU NL SE**

(56) Documents cité:
**FR-A-1 545 142**

**HOUBEN-WEYL: METHODEN DER ORGANISCHEN CHEMIE, vol. E4: Kohlensäure-Derivate, 1983, pages 36-38 et 768-770, Georg Thieme Verlag, Stuttgart, DE;**
**HOUBEN-WEYL: METHODEN DER ORGANISCHEN CHEMIE, vol. V/3: Halogenverbindungen, 1962, pages 119-124, Georg Thieme Verlag, Stuttgart, DE;**

**Le dossier contient des informations techniques présentées postérieurement au dépôt de la demande et ne figurant pas dans le présent fascicule.**

(73) Titulaire: **RHONE- POULENC SPECIALITES CHIMIQUES, "Les Miroirs" 18, Avenue d'Alsace, F-92400 Courbevoie (FR)**

(72) Inventeur: **Desbois, Michel, 526 Chemin du Bois, F-69140 - Rillieux (FR)**

(74) Mandataire: **Cazes, Jean- Marie, RHONE- POULENC INTERSERVICES Service Brevets Chimie 25, quai Paul Doumer, F-92408 Courbevoie Cédex (FR)**

**EP 0 152 310 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne un procédé de préparation de trifluorométhyl anilines.

Elle concerne plus particulièrement un procédé de préparation de para trifluorométhyl anilines.

Il est connu de préparer la paratrifluorométhylaniline à partir du p-chlorotrifluorométhylbenzène par ammonolyse selon le procédé de SEIWELL (J. Org. Chem. 1979, 44 (25), 4731-3) dans un solvant organique à l'aide de chlorure cuivreux et de fluorure de potassium. Le taux de conversion et le rendement en p-trifluorométhylaniline sont faibles, ce procédé est donc difficilement exploitable industriellement.

Sont également connus de nombreux procédés J. Org. Chem. 26, 1477-80 (1961), J. Amer. Chem. Soc. 69, 2346-50 (1947) partant du p-nitrotrifluorométhylbenzène sur lequel est réalisée une réduction catalytique. Mais l'accès au p-nitrotrifluorométhylbenzène est très difficile, cette méthode n'est donc pas utilisable industriellement.

Il existe aussi un procédé de préparation de trifluorométhylaniline, selon le brevet français N° 1 545 142, à partir de trifluorométhylisocyanates par hydrolyse en milieu acide sulfurique. Pour obtenir le trifluorométhylphénylisocyanate, on doit procéder à une fluoration en milieu acide fluorhydrique du trichlorométhylphénylisocyanate puis à une extraction difficile du milieu HF pour transférer le trifluorométhylphénylisocyanate dans le milieu $H_2SO_4$ où a lieu l'hydrolyse.

Il est maintenant possible de réaliser la préparation en une seule étape et avec d'excellents rendements de para trifluorométhyl anilines à partir d'isocyanates benzéniques ou d'halogénures de carbamoyles para trihalogénométhylés.

En effet, la présente invention concerne un procédé de préparation de para trifluorométhyl anilines caractérisé en ce que l'on effectue simultanément l'hydrolyse et la fluoration échange d'un isocyanate benzénique ou d'un halogénure de carbamoyle porteur d'un groupement trihalogénométhyle en position para dans de l'acide fluorhydrique liquide.

On entend par fluoration échange, le fait d'échanger les halogènes du groupe trihalogénométhyle par des atomes de fluor. De préférence, l'isocyanate benzénique ou l'halogénure de carbamoyle répond à la formule générale (I) suivante :

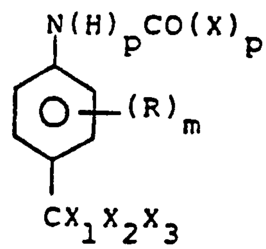

dans laquelle:

- p est égal à 0 ou 1, X étant un halogène
- $X_1$ $X_2$ $X_3$ représentent chacun un atome d'halogène identique ou différent, l'un au moins n'étant pas le fluor
- R est un groupe inerte dans les conditions de la réaction ; il est de préférence choisi parmi le groupe comprenant l'hydrogène et les radicaux halogéno, alkyle comprenant de 1 à 4 atomes de carbone alkoxy, alkylthio, hydroxyle, carboxyle, nitro, carbonyle, cyano - m est égal à 1 ou 2.

Si $p = 0$, la formule (I) représente un isocyanate et si $p = 1$, la formule (I) représente un halogénure de carbamoyle.

Le procédé est tout particulièrement adapté aux composés de formule (I) où R est l'hydrogène et $p = 0$ comme par exemple à l'isocyanate de para trichlorométhylphényle car la paratrifluorométhylaniline obtenue dans ce cas est un intermédiaire de synthèse très utilisé dans l'industrie pharmaceutique et phytosanitaire. L'hydrolyse réalisée en milieu fluorhydrique minimise fortement la formation d'urée.

Le fait d'utiliser l'acide fluorhydrique permet un recyclage de ce dernier ce qui n'est pas possible lorsque l'on utilise comme dans le brevet français N° 1 545 142 l'acide sulfurique ; il évite d'autre part un changement de réacteur dû aux deux étapes du procédé et élimine les problèmes techniques occasionnés par la défluoration en milieu sulfurique.

L'acide fluorhydrique utilisé contient de préférence, pour permettre l'hydrolyse dans les meilleurs conditions, une quantité d'eau telle que le rapport molaire de l'eau au composé (I) soit compris entre 1 et 2.

Il est toutefois encore plus préférable d'utiliser une quantité d'eau telle que le rapport molaire de l'eau au composé de formule (I) soit égal à environ 1.

L'acide fluorhydrique servant de milieu réactionnel, la quantité minimale dervrait de préférence permettre de dissoudre l'isocyanate, la quantité maximale n'est pas critique, elle sera simplement adaptée à l'économie du procédé. Il est néanmoins économiquement et industriellement préférable d'utiliser un rapport molaire de l'acide fluorhydrique au composé de formule (I) compris entre 5 et 50.

La réaction est réalisée à une température comprise de préférence entre 20 et 80°C.

Lorsque l'hydrolyse sera réalisée à une température supérieure à 20°C, la réaction devra avoir lieu sous pression puisque l'acide fluorhydrique doit être liquide.

La durée de la réaction varie de quelques minutes à quelques heures. Cette durée de réaction varie avec les

matières premières de départ et avec la température de réaction.

L'aniline obtenue est isolée de manière connue en soi. Par exemple on élimine par distillation l'acide fluorhydrique qui ainsi est récupérable et recyclable ce qui est un avantage important du procédé de l'invention puis on neutralise le sel obtenu par une base et on distille l'aniline ainsi obtenue.

On peut citer comme exemple de produits pouvant être mis en oeuvre selon le procédé de l'invention : le trichlorométhyl-4 phénylisocyanate, le chlorure de carbamoyle de la trichlorométhyl-4 aniline; le tribromométhyl-4 phényl isocyanate, le bromure de carbamoyle de la tribromométhyl-4 aniline, le trichlorométhyl-4 chloro-3 phénylisocyanate, le trichlorométhyl-4 nitro-3 phénylisocyanate, le trichlorométhyl-4 dichloro-3,5 phénylisocyanate, le trichloro-méthyl-4 méthylcarbonyl-3 phénylisocyanate, le trichlorométhyl-4 isopropyl-3 phénylisocyanate.

Les trifluorométhyl anilines obtenues par le procédé de l'invention répondent à la formule générale (II) suivante

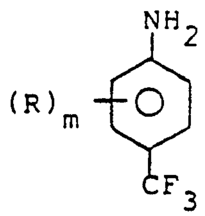

(II)

dans laquelle R a la signification précédente.

On peut citer parmi les anilines répondant à la formule (II) la paratrifluorométhylaniline.

Les anilines obtenues par le procédé de la présente invention sont utilisées comme intermédiaire de synthèse dans l'industrie pharmaceutique, phytosanitaire ou pour la synthèse de colorants (brevets US 3 463 787 et BF 1 520 220).

La présente invention sera plus aisément comprise à l'aide des exemples suivants, donnés à titre indicatif mais non limitatif.

**Exemple 1** - Chloro-3 amino-4 trifluorométhylbenzène

Dans un réacteur inox de 250 ml agité par un barreau aimanté et refroidi aux environs de 0°C, on introduit sous agitation successivement 100 g (5 m) de HF anhydre, 3,6 g (0,2 m) d'eau et 54,2 g (0,2 m) de chloro-2 trichlorométhyl-4 phénylisocyanate dont la préparation peut se faire par chloration ($Cl_2$) in situ du trichlorométhyl-4 phénylisocyanate. Le réacteur est fermé puis porté à 50°C pendant 3 H 30. Après refroidissement, le mélange brut réactionnel est coulé sur 200 g de glace pilée puis neutralisé jusqu'à pH $\cong$ 10 par une solution de potasse 50 % en ne dépassant pas la température de 20°C.

On extrait alors cette phase aqueuse par 3 fois 100 ml de chlorure de méthylène. Les phases organiques sont rassemblées, séchées puis évaporées sous pression réduite. On recueille environ 31,5 g d'un composé liquide essentiellement constitué de chloro-3 amino-4 trifluorométhylbenzène - Analyses chromatographie phase gazeuse (CPG), infrarouge (IR) et Masse -.

**Exemple 2** - Amino-4 trifluorométhylbenzène

On opère de manière identique à l'exemple 1 avec les composés et conditions suivants:

| | |
|---|---|
| - Acide fluorhydrique | 100 g (5 m) |
| - Eau | 1,8 g (0,1 m) |
| - Trichlorométhyl-4 phénylisocyanate | 23,6 g (0,1 m) |
| - Température | 60°C |
| - Durée | 5 heures |

On recueille 12,9 g d'un composé liquide essentiellement constitué de p-trifluorométhylaniline.

**Exemple 3** - Nitro-3 amino-4 trifluorométhylbenzène

On opère de manière identique à l'exemple 1 avec les composés et conditions suivants :

| | |
|---|---|
| - Acide fluorhydrique | 100 g (5 m) |
| - Eau | 9 g (0,4 m) |
| - Nitro-2 trichlorométhyl-4 phénylisocyanate | 125 g (0,4 m) |
| - Température | 80°C |
| - Durée | 6 heures |

On recueille 56 g d'un composé liquide essentiellement constitué de nitro-3 amino-4 trifluorométhylbenzène.

**Revendications**

pour les Etats contractants AT, BE, CH, LI, LU, SE

1. Procédé de préparation de para trifluorométhyl anilines caractérisé en ce qu'on effectue simultanément l'hydrolyse et la fluoration échange d'un isocyanate benzénique ou d'un halogénure de carbamoyle para trihalogénométhylé dans de l'acide fluorhydrique liquide.

2. Procédé selon la revendication 1 caractérisé en ce que l'isocyanate ou l'halogénure de carbamoyle répond à la formule générale suivante :

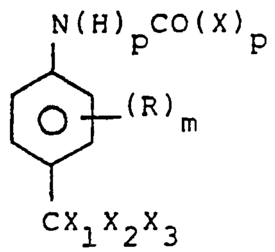

(I)

dans laquelle
- X est un halogène
- p est égal à 0 ou 1,
- $X_1$ $X_2$ $X_3$ représentent chacun un atome d'halogène identique ou différent l'un au moins n'étant pas le fluor
- R est choisi parmi le groupe comprenant l'hydrogène et les radicaux halogéno, alkyle comprenant de 1 à 4 atomes de carbone, alkoxy, alkylthio, hydroxyle, carboxyle, nitro
- m est égal à 1 ou 2.

3. Procédé selon l'une quelconque des revendications 1 et 2 caractérisé en ce que dans la formule I, R représente l'hydrogène et p est égal à 0.

4. Procédé selon la revendication 3 caractérisé en ce que le composé de formule (I) est le para trichlorométhylbenzène isocyanate.

5. Procédé selon la revendication 1 caractérisé en ce que l'acide fluorhydrique contient une quantité d'eau telle que le rapport molaire de l'eau au composé de formule (I) est compris entre 1 et 2.

6. Procédé selon la revendication 5 caractérisé en ce que l'acide fluorhydrique contient une quantité d'eau telle que le rapport molaire de l'eau au composé de formule (I) est égal à 1.

7. Procédé selon la revendication 1 caractérisé en ce que le rapport molaire acide fluorhydrique au composé de formule (I) est compris entre 5 et 50.

8. Procédé selon la revendication 1 caractérisé en ce que la température de réaction est comprise entre 20 et 80°C.

**Revendications**

pour les Etats contractants: NL, DE, FR, GB, I.

1. Procédé de préparation de para trifluorométhyl anilines caractérisé en ce qu'on effectue simultanément l'hydrolyse et la fluoration échange d'un isocyanate benzénique ou d'un halogénure de carbamoyle para trihalogénométylé dans de l'acide fluorhydrique liquide à une température d'au minimum 50°C et à pression autogène.

2. Procédé selon la revendication 1, caractérisé en ce que l'isocyanate ou l'halogénure de carbamoyle répond à la formule générale suivante:

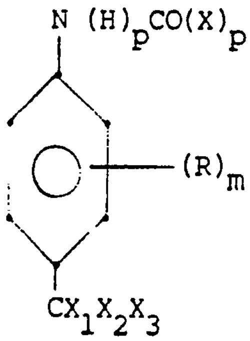

(I)

dans laquelle:
- X est un halogène
- p est égal à 0 ou 1,
- $X_1$ $X_2$ $X_3$ représentent chacun un atome d'halogène identique ou différent l'un au moins n'étant pas le fluor.
- R est choisi parmi le groupe comprenant l'hydrogène et les radicaux halogéno, alkyle comprenant de 1 à 4 atomes de carbone alkoxy, alkylthio, hydroxyle, carboxyle, nitro.
- m est égal à 1 ou 2.

3. Procédé selon l'une quelconque des revendications 1 et 2 caractérisé en ce que dans la formule I, R représente l'hydrogène et p est égal à 0.

4. Procédé selon la revendication 3 caractérisé en ce que le composé de formule (I) est le para trichlorométhylbenzène isocyanate.

5. Procédé selon la revendication 1 caractérisé en ce que l'acide fluorhydrique contient une quantité d'eau telle que le rapport molaire de l'eau au composé de formule (I) est compris entre 1 et 2.

6. Procédé selon la revendication 5 caractérisé en ce que l'acide fluorhydrique contient une quantité d'eau telle que le rapport molaire de l'eau au composé de formule (I) est égal à 1.

7. Procédé selon la revendication 1 caractérisé en ce que le rapport molaire acide fluorhydrique au composé de formule (I) est compris entre 5 et 50.

8. Procédé selon la revendication 1 caractérisé en ce que la température de réaction est comprise entre 50 et 80°c.

**Patentansprüche**

für die Vertragsstaaten AT, BE, CH, LI, LU, SE

1. Verfahren zur Herstellung von para-Trifluormethylanilinen, dadurch gekennzeichnet, daß man gleichzeitig die Hydrolyse und die Austauschfluorierung eines Benzolisocyanats oder eines (para-Trihalogenomethyl)carbamoylhalogenids in flüssiger Fluorwasserstoffsäure durchführt.

2. Verfahren nach Anspruch 1, dadurch gekennzeichnet, daß das Isocyanat oder das Carbamoylhalogenid der nachfolgenden allgemeinen Formel entspricht:

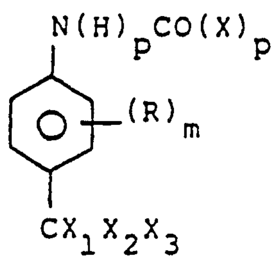

(I)

in der:
- X ein Halogen ist
- p gleich 0 oder 1 ist,
- $X_1$ $X_2$ $X_3$ jeweils gleiche oder unterschiedliche Halogenatome darstellen, wobei mindestens eines von diesen nicht Fluor ist
- R ausgewählt wird aus der Gruppe bestehend aus Wasserstoff und den Resten Halogen, Alkyl mit 1 bis 4 Kohlenstoffatomen, Alkoxy, Alkylthio, Hydroxyl, Carboxyl, Nitro
- m gleich 1 oder 2 ist.

3. Verfahren nach einem der Ansprüche 1 und 2, dadurch gekennzeichnet, daß in der Formel I R ein Wasserstoffatom darstellt und p gleich 0 ist.

4. Verfahren nach Anspruch 3, dadurch gekennzeichnet, daß die Verbindung gemäß Formel I das para-Trichlormethylbenzolisocyanat ist.

5. Verfahren nach Anspruch 1, dadurch gekennzeichnet, daß die Fluorwasserstoffsäure eine solche Menge Wasser enthält, daß das molare Verhältnis von Wasser zur Verbindung der Formel (I) zwischen 1 und 2 liegt.

6. Verfahren nach Anspruch 5, dadurch gekennzeichnet, daß die Fluorwasserstoffsäure eine solche Menge Wasser enthält, daß das molare Verhältnis von Wasser zur Verbindung der Formel (I) gleich 1 ist.

7. Verfahren nach Anspruch 1, dadurch gekennzeichnet, daß das molare Verhältnis Fluorwasserstoffsäure zur Verbindung der Formel I zwischen 5 und 50 liegt.

8. Verfahren nach Anspruch 1, dadurch gekennzeichnet, daß die Reaktionstemperatur zwischen 50° und 80°C liegt.

**Patentansprüche**

für die Vertragsstaaten NL, DE, FR, GB, IT

1. Verfahren zur Herstellung von para-Trifluormethylanilinen, dadurch gekennzeichnet, daß man gleichzeitig die Hydrolyse und die Austauschfluorierung eines Benzolisocyanats oder eines (para-Trihalogenomethyl)carbamoylhalogenids in flüssiger Fluorwasserstoffsäure bei einer Temperatur von von mindestens 50°C und autogenem Druck durchführt.

2. Verfahren nach Anspruch 1, dadurch gekennzeichnet, daß das Isocyanat oder das Carbamoylhalogenid der nachfolgenden allgemeinen Formel entspricht.:

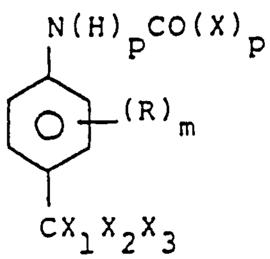

(I)

in der:

- X ein Halogen ist
- p gleich 0 oder 1 ist,
- $X_1$ $X_2$ $X_3$ jeweils Halogenatome darstellen, von denen mindestens eines nicht Fluor ist
- R ausgewählt wird aus der Gruppe umfassend Wasserstoff und den Resten Halogen, Alkyl mit 1 bis 4 Kohlenstoffatomen, Alkoxy, Alkylthio,Hydroxyl, Carboxyl, Nitro
- m gleich 1 oder 2 ist.

3. Verfahren nach einem der Ansprüche 1 und 2, dadurch gekennzeichnet, daß in der Formel I R ein Wasserstoffatom darstellt und p gleich 0 ist.

4. Verfahren nach Anspruch 3, dadurch gekennzeichnet, daß die Verbindung der Formel I das para-Trichlormethylbenzolisocyanat ist.

5. Verfahren nach Anspruch 1, dadurch gekennzeichnet, daß die Fluorwasserstoffsäure eine solche Menge Wasser enthält, daß das molare Verhältnis von Wasser zur Verbindung der Formel (I) zwischen 1 und 2 liegt.

6. Verfahren nach Anspruch 5, dadurch gekennzeichnet, daß die Fluorwasserstoffsäure eine solche Menge Wasser enthält, daß das molare Verhältnis von Wasser zur Verbindung der Formel (I) gleich 1 ist.

7. Verfahren nach Anspruch 1, dadurch gekennzeichnet, daß das molare Verhältnis von Fluorwasserstoffsäure zur Verbindung der Formel I zwischen 5 und 50 liegt.

8. Verfahren nach Anspruch 1, dadurch gekennzeichnet, daß die Reaktionstemperatur zwischen 50° und 80°C liegt.

**Claims**

for the contracting States AT, BE, CH, LI, LU, SE

1. Process for preparing para-trifluoromethylanilines, characterized in that the hydrolysis and the exchange fluoridation of a benzenoid isocyanate or a paratrihalomethyl-containing carbamoyl halide are performed simultaneously in liquid hydrofluoric acid.

2. Process according to Claim 1, characterized in that the isocyanate or carbamoyl halide corresponds to the

following general formula:

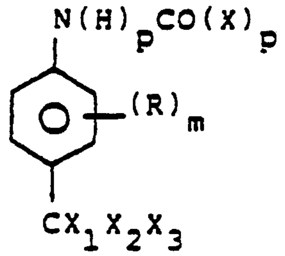

(I)

in which:
X is a halogen,
p is equal to 0 or 1,
$X_1$, $X_2$, $X_3$ each denote an identical or different halogen atom, at least one not being fluorine,
R is chosen from the group comprising hydrogen and halo radicals, alkyl radicals comprising from 1 to 4 carbon atoms, and alkoxy, alkylthio, hydroxyl, carboxyl and nitro radicals, and
m is equal to 1 or 2.

3. Process according to either of Claims 1 and 2, characterized in that, in the formula I, R denotes hydrogen and p is equal to 0.

4. Process according to Claim 3, characterized in that the compound of formula (I) is para-trichloromethylphenyl isocyanate.

5. Process according to Claim 1, characterized in that the hydrofluoric acid contains an amount of water such that the mole ratio of water to the compound of formula (I) is between 1 and 2.

6. Process according to Claim 5, characterized in that the hydrofluoric acid contains an amount of water such that the mole ratio of water to the compound of formula (I) is equal to 1.

7. Process according to Claim 1, characterized in that the mole ratio of hydrofluoric acid to the compound of formula (I) is between 5 and 50.

8. Process according to Claim 1, characterized in that the reaction temperature is between 20 and 80°C.

## Claims

for the contracting States NL, DE, FR, GB, IT

1. Process for preparing para-trifluoromethylanilines, characterized in that the hydrolysis and the exchange fluoridation of a benzenoid isocyanate or a paratrihalomethyl-containing carbamoyl halide are performed simultaneously in liquid hydrofluoric acid at a temperature of not less than 50°C and at the self-generated pressure.

2. Process according to Claim 1, characterized in that the isocyanate or carbamoyl halide corresponds to the following general formula:.

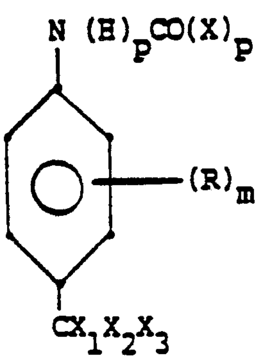

(I)

in which:
X is a halogen,
p is equal to 0 or 1,
$X_1$, $X_2$, $X_3$ each denote an identical or different halogen atom, at least one not being fluorine,
R is chosen from the group comprising hydrogen and halo radicals, alkyl radicals comprising from 1 to 4 carbon atoms, and alkoxy, alkylthio, hydroxyl, carboxyl and nitro radicals, and
m is equal to 1 or 2.

3. Process according to either of Claims 1 and 2, characterized in that, in the formula I, R denotes hydrogen and p is equal to 0.

4. Process according to Claim 3, characterized in that the compound of formula (I) is para-trichloromethylphenyl isocyanate.

5. Process according to Claim 1, characterized in that the hydrofluoric acid contains an amount of water such that the mole ratio of water to the compound of formula (I) is between 1 and 2.

6. Process according to Claim 5, characterized in that the hydrofluoric acid contains an amount of water such that the mole ratio of water to the compound of formula (I) is equal to 1.

7. Process according to Claim 1, characterized in that the mole ratio of hydrofluoric acid to the compound of formula (I) is between 5 and 50.

8. Process according to Claim 1, characterized in that the reaction temperature is between 50 and 80° C.